# DEMANDE DE BREVET EUROPEEN

(11) **EP 1 958 653 A1**
(43) Date de publication de la demande: **20.08.2008**
(21) Numéro de dépôt: 07405043.6
(22) Date de dépôt: 14.02.2007
(51) Int. Cl.: A61M 5/142, B04B 5/04, B65D 47/00, F04B 43/00, H01R 13/00

(54) **Enceinte étanche et procédé de fabrication de cette enceinte**

(71) Demandeur: Rochat, Jean-Denis, 1272 Genolier (CH)
(72) Inventeur: Rochat, Jean-Davis, 1272 Genolier (CH)
(74) Mandataire: Savoye, Jean-Paul

(57) **Abrégé**

Enceinte étanche dont la paroi est en deux parties (1, 3) qui présentent des surfaces périphériques d'assemblage homologues respectives (1a, 3a), réunies de manière étanche l'une à l'autre, cette enceinte comportant au moins une ouverture (6, 7) pour la relier avec l'extérieur et une partie intermédiaire (2) disposée entre les deux parties de paroi (1, 3) de l'enceinte. Cette enceinte forme initialement une pièce unique avec la partie intermédiaire (2) disposée entre les surfaces périphériques d'assemblage homologues (1a, 3a) des deux parties de paroi (1, 3), cette pièce unique comportant deux liaisons d'articulation (4, 5), entre deux portions opposées de la périphérie de ladite partie intermédiaire (2) et respectivement les surfaces périphériques d'assemblage homologues (1a) des deux parties (1, 3) de la paroi de l'enceinte étanche.

## Description

La présente invention se rapporte à une enceinte étanche dont la paroi est en deux parties qui présentent des surfaces périphériques d'assemblage homologues respectives, réunies de manière étanche l'une à l'autre, cette enceinte comportant au moins une ouverture pour la relier avec l'extérieur et une partie intermédiaire disposée entre les deux parties de paroi de l'enceinte, ainsi qu'à un procédé de fabrication de cette enceinte.

On connaît des structures de ce type dans lesquelles la cloison intermédiaire peut comporter un filtre, les ouvertures disposées de part et d'autre du filtre étant reliées à un conduit d'écoulement d'un liquide; un circuit électronique, l'enceinte servant à protéger ce circuit du milieu environnant. La cloison intermédiaire peut aussi présenter une ouverture contrôlée par un clapet, ainsi qu'un clapet pour contrôler l'ouverture d'admission, une portion au moins de la paroi de l'enceinte étant déformable pour permettre de créer alternativement une dépression et une surpression dans l'enceinte apte à faire circuler un liquide dans un conduit auquel cette enceinte étanche est reliée.

Actuellement, une telle enceinte, typiquement en matière plastique, nécessite la fabrication de trois pièces injectées. La prise de ces pièces tombées en vrac à la sortie des moules par trois robots (un par pièce), l'assemblage des pièces et leur soudage aux ultrasons.

Un champ d'application privilégié de telles enceintes se situe dans le domaine des produits consommables à usage unique dans le domaine médical, où la réutilisation est exclue en raison des exigences relatives à la stérilité du matériel utilisé. Dès lors, la réduction du coût de ce matériel consommable à usage unique prend une grande importance.

Le but de la présente invention est de modifier la structure d'une telle enceinte afin de simplifier sensiblement son procédé de fabrication et d'abaisser le coût de production.

A cet effet, cette invention a tout d'abord pour objet une enceinte étanche selon la revendication 1. Elle a également pour objet un procédé de fabrication de cette enceinte selon la revendication 8.

Cette enceinte et son procédé de fabrication présentent plusieurs avantages, parmi lesquels on peut citer le fait de remplacer trois pièces moulées séparément par une pièce unique, le fait de réunir ces trois pièces par des liaisons d'articulation et celui de disposer les deux parties de l'enceinte tête-bêche dans le moule et de les relier par la cloison intermédiaire, en sorte qu'un mouvement de translation d'une des deux parties selon une trajectoire semi-circulaire en prenant les liaisons d'articulation comme centres de rotation permet de réunir les surfaces périphériques d'assemblage complémentaires respectives des deux parties de l'enceinte et la périphérie de la cloison intermédiaire. Comme on peut s'en rendre compte, la conception de cette enceinte et son procédé de fabrication, permettent de réduire de manière significative le coût de production.

D'autres particularités et avantages apparaîtront au cours de la description suivante de deux formes d'exécution d'une enceinte étanche et de son processus de fabrication illustrés, schématiquement et à titre d'exemple par les dessins annexés, dans lesquels :
les figures 1, 1a sont des vue en perspective d'une première forme d'exécution d'une enceinte étanche en cours de formation, la figure 1a montrant un détail agrandi de la figure 1;
la figure 2 est une vue en coupe d'un moule montrant une première étape du processus de fabrication de l'enceinte étanche illustrée par la figure 1;
la figure 3 est une vue semblable à la figure 2 montrant une deuxième étape du processus de fabrication;
la figure 4 est une vue en élévation de l'enceinte étanche obtenue par le processus illustré par les figures 2 et 3;
la figure 5 est une vue en coupe et en perspective d'une seconde forme d'exécution d'une enceinte étanche formant une pompe en cours de formation;
la figure 6 est une vue en élévation en coupe de la pompe de la figure 5 terminée.

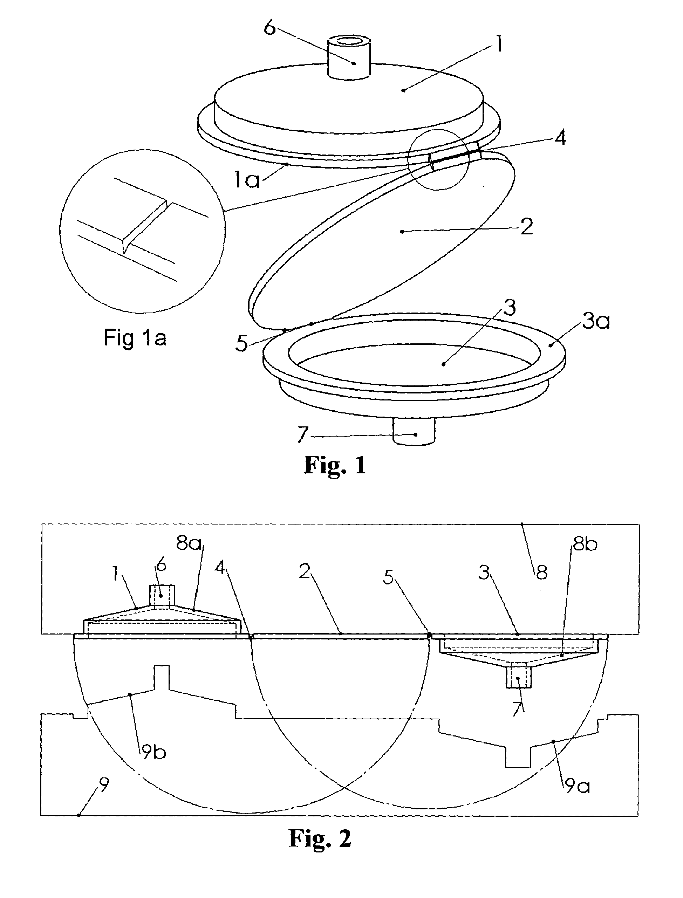

L'enceinte étanche, dans cet exemple en matière plastique injectée, illustrée par la figure 1 au cours de sa formation, comporte trois parties 1, 2, 3, reliées les unes aux autres par deux liaisons d'articulation 4, 5 dont l'une 4 est illustrée à plus grande échelle par la figure 1a. Les parties 1 et 3 sont des parties de la paroi de l'enceinte. Ces parties de paroi 1 et 3 comportent des surfaces périphériques d'assemblage homologues 1a, respectivement 3a. La partie 2 est une partie intermédiaire reliée par les liaisons d'articulation 4, 5 situées dans deux portions opposées de la périphérie de la partie 2, aux deux parties de paroi 1, respectivement 3. La périphérie de la partie intermédiaire 2 correspond aux surfaces périphériques d'assemblage complémentaires 1a, 3a des parties de paroi 1, respectivement 3 et est destinée à venir entre ces surfaces d'assemblage complémentaires 1a, 3a une fois le pliage de la structure de l'enceinte terminé.

Ces trois parties 1, 2, 3 de l'enceinte étanche sont fixées l'une à l'autre par soudage aux ultrasons de leurs surfaces d'assemblage périphériques homologues 1a, 3a avec le bord périphérique de la partie intermédiaire 2.

Dans cet exemple, chaque partie de paroi 1, 3 de l'enceinte étanche comporte encore une ouverture 6, respectivement 7 pour faire communiquer l'intérieur de l'enceinte avec l'extérieur. Cette ouverture peut être utilisée à différentes fins en fonction de la nature de l'enceinte, pour faire circuler un fluide, en particulier un liquide, dans le cas où la partie intermédiaire porte ou constitue elle-même un filtre, mais aussi pour faire passer des conducteurs électriques, dans le cas où la partie intermédiaire 2 est utilisée comme support d'un circuit électronique par exemple. Dans ce cas, une seule ouverture est généralement suffisante. On peut aussi envisager le cas où deux ouvertures sont disposées dans la même partie de paroi 1 ou 3.

Les figures 2 à 4 illustrent les différentes étapes du procédé de fabrication de l'enceinte étanche décrite ci-dessus.

L'étape 1 consiste à injecter en une seule pièce les parties 1, 2, 3 de l'enceinte, reliées les unes aux autres par les liaisons d'articulation 4, 5. Cette pièce est obtenue dans un moule en deux pièces 8 et 9, en ménageant dans chacune d'elles une empreinte 8a, respectivement 9a de la face externe des deux parties de paroi 1, 3 de l'enceinte et une empreinte 8b, respectivement 9b de la face interne de ces mêmes deux parties de paroi 1, 3 de l'enceinte et on relie ces deux empreintes par une empreinte de la partie intermédiaire 2 et des liaisons d'articulation 4, 5, en sorte que l'on obtient, après refroidissement, une pièce moulée par injection dont les deux parties de paroi 1, 3 de l'enceinte sont tête-bêche avec interposition de la partie intermédiaire 2.

Grâce à cette disposition tête-bêche des parties de paroi 1, 3 de part et d'autre de la partie intermédiaire 2, un robot (non représenté) peut saisir la partie de paroi 3 par une portion de conduit cylindrique traversé par l'ouverture 7 pour faire communiquer l'enceinte étanche avec l'extérieur et lui faire subir un mouvement de translation selon une trajectoire semi-circulaire illustrée par les demi-cercles en traits mixtes de la figure 2, dont le rayon correspond à la distance séparant les liaisons d'articulation 4, 5.

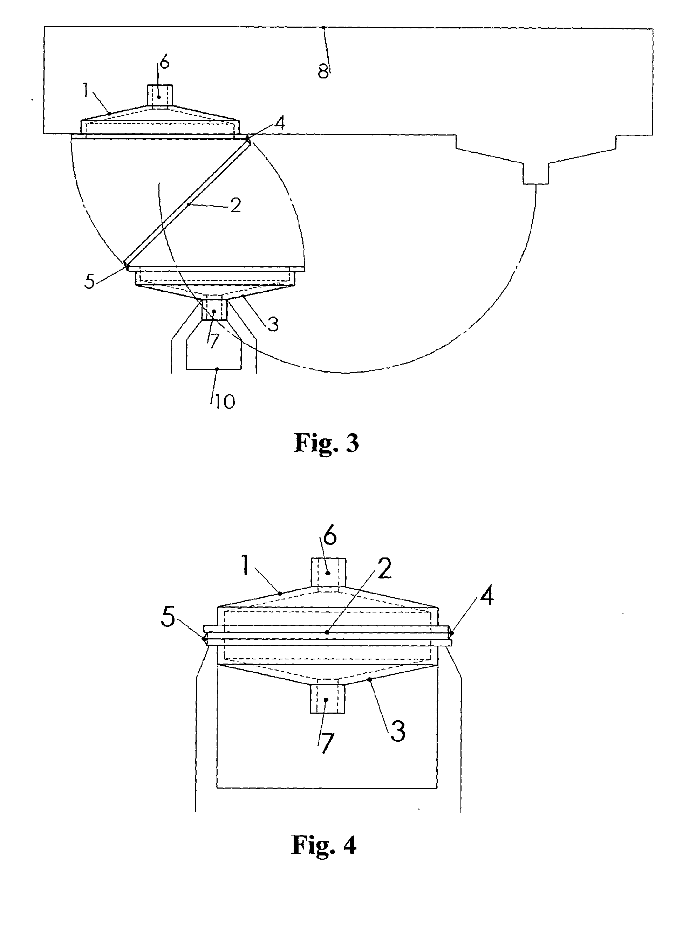

La figure 3 montre les parties 1, 2, 3 de l'enceinte étanche au cours de leur déplacement en translation selon une trajectoire semi-circulaire, la partie 1 restant fixe dans l'empreinte de moulage 8a de sa face externe. Ce mouvement est possible grâce aux liaisons d'articulation 4, 5.

La figure 4 montre la fin du mouvement de translation avec les surfaces d'assemblage périphériques homologues respectives 1a, 3a qui viennent en appui de part et d'autre du bord de la partie intermédiaire 2. Ces surfaces d'assemblage 1a, 3a sont avantageusement tout d'abord assemblées par clipage puis soudées par ultrasons à la périphérie de la partie intermédiaire 2. Le soudage est réalisé sur le moule même par un robot pénétrant dans le moule. Ce procédé permet d'éviter les reprises pour le soudage.

La partie intermédiaire peut constituer un support d'un circuit électronique, ou d'un dispositif qui doit être protégé par l'enceinte étanche. Les ouvertures 6, 7 peuvent aussi servir à relier ce boîtier à une circulation de fluide ou de liquide, en particulier dans le domaine médical. Dans ce cas, la partie intermédiaire peut être formée pour jouer le rôle de filtre ou de support de filtre, auquel cas, ce filtre devrait être placé sur le support constitué par la partie intermédiaire 2 après l'ouverture du moule, à l'étape illustrée par la figure 2. Il devrait en être de même au cas où la partie intermédiaire serait constituée par un circuit électronique ou par un dispositif à protéger dans l'enceinte étanche.

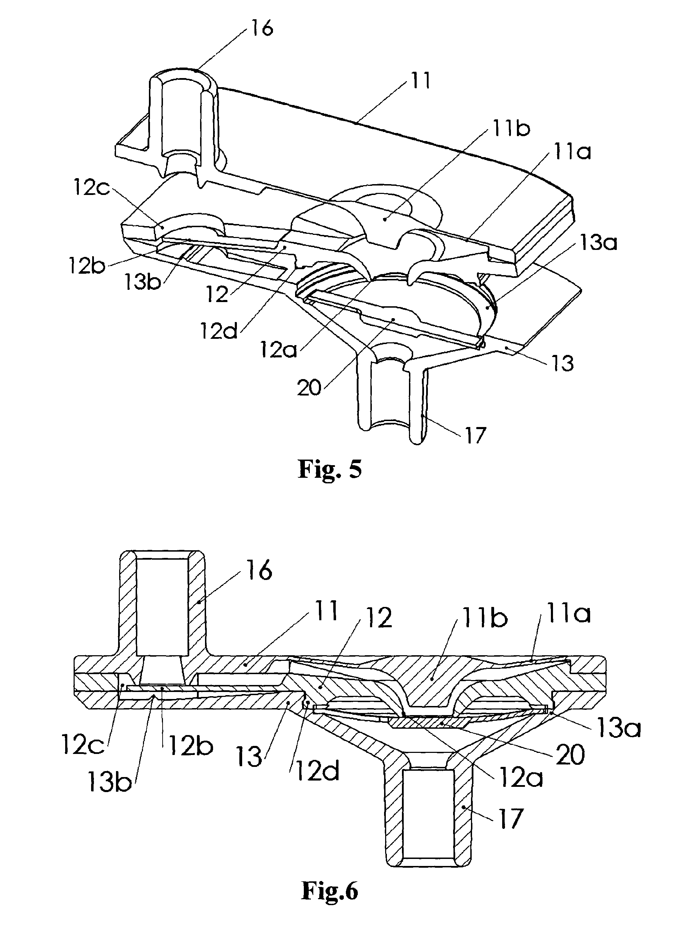

Les figure 5 et 6 illustrent une forme d'exécution de l'enceinte objet de la présente invention, dans laquelle cette enceinte constitue une pompe, en particulier une pompe jetable utilisée dans le domaine médical.

Comme on peut le constater, cette pompe est essentiellement formée d'une enceinte en trois parties 11, 12, 13, deux parties 11, 13 formant la paroi de l'enceinte de pompage et une partie intermédiaire 12. Ces trois parties 11, 12, 13 sont reliées les unes aux autres par deux liaisons d'articulation 14, 15. Chacune des parties de paroi 11, 13 comporte un conduit d'admission 16 et un conduit de refoulement 17. Les différences entre cette forme d'exécution et celle décrite aux figures 1 à 4 résident essentiellement, au niveau de la structure, dans le fait que la partie de paroi 11 présente une partie amincie formant une membrane annulaire 11a, entourant une partie d'actionnement épaisse 11b. La partie annulaire amincie 11a sert de membrane de pompage dont la partie centrale d'actionnement épaisse 11b sert à avec transmettre à la membrane annulaire la force exercée par un poussoir (non représenté) formé avantageusement par le noyau mobile d'un électro-aimant d'entraînement de la pompe. La déformation de la membrane doit évidemment rester dans les limites de déformation élastique de la matière plastique formant la partie de paroi 11.

La partie intermédiaire 12 comporte une ouverture de communication 12a pour mettre en communication sélective les compartiments amont et aval de la pompe. Cette ouverture de communication 12a est disposée en regard de la partie centrale épaisse 11b de la membrane et un clapet de valve 12b situé en regard d'une ouverture 12c vis-à-vis de l'extrémité interne du conduit d'admission 16 ménagé dans la partie de paroi 11. Comme illustré sur les figures 5 et 6, l'ouverture 12a se situe à l'extrémité d'une dépression annulaire incurvée, à face convexe, tandis que la partie centrale d'actionnement épaisse 11b forme une saillie à face incurvée concave, complémentaire de celle de la dépression annulaire percée de l'ouverture 12a. La partie intermédiaire 12 comporte encore une saillie annulaire 12d tournée vers la partie de paroi 13, dont le rôle sera expliqué ci-après.

La partie de paroi 13 comporte un siège concentrique au conduit de refoulement 17, de positionnement d'un clapet 20 de contrôle de l'ouverture de communication 12a de la partie intermédiaire. Ce clapet 20 est disposé entre cette ouverture de communication 12a et le conduit de refoulement 17 de la pompe. En position de repos, il ferme l'ouverture 12a et il est appliqué contre celle-ci dès que la pression qui règne en amont de l'ouverture de communication 12a est inférieure à celle qui règne en aval de cette même ouverture de communication 12a. Elle s'écarte de cette ouverture 12a dès que les pressions sus-mentionnées respectives s'inversent.

La partie de paroi 13 présente un siège annulaire 13a pour le positionnement du clapet 20. Ce clapet 20 est retenu sur ce siège 13a par la saillie annulaire 12d de la partie intermédiaire 12. La partie de paroi 13 comporte encore une saillie 13b située en face du clapet 12b de contrôle du conduit d'admission 16, pour empêcher que ce clapet 12b ne vienne s'appliquer contre la face interne de la paroi de la partie de paroi 13, pour permettre d'exposer la face de ce clapet 12b opposée à sa face adjacente à l'extrémité interne du conduit d'admission 16 de la pompe à la pression régnant dans le compartiment de la pompe situé en amont de l'ouverture de communication 12a de la partie intermédiaire 12 et permettre à ce clapet 12b de fermer l'extrémité interne du conduit d'admission 16 en phase de refoulement de la pompe et de s'ouvrir en phase d'aspiration.

Le procédé de fabrication de cette pompe ne diffère de celui de la forme d'exécution précédente que par le fait que lors du moulage par injection des parties 11, 12, 13, on injecte dans le même moule, mais en tant qu'élément séparé, le clapet 20. Après avoir ouvert le moule, comme illustré à la figure 2 de la forme d'exécution précédente, on trouve donc deux pièces séparées, la pièce formée de parties 11, 12, 13 reliées entre elles par les liaisons d'articulation 14, 15 et le clapet 20.

Avant de commencer le pliage en Z des trois parties 11, 12, 13, un robot prend le clapet 20 et vient le placer sur le siège 13a de la partie de paroi 13. Dans ce cas, c'est donc cette partie de paroi 13 qui doit rester fixe dans l'empreinte du moule. et les parties 11 et 12 qui doivent être déplacées selon une translation pour former un Z qui s'écrase progressivement. La dernière opération consiste de nouveau dans l'assemblage par clipage et la fixation par soudage aux ultrasons des surfaces périphériques d'assemblage homologues respectives des trois parties 11, 12, 13.

On peut constater à la lecture des exemples qui précèdent que la structure de l'enceinte décrite et son procédé de fabrication permettent de réduire les coûts de production à un minimum. En effet, le nombre de pièces séparées est réduit à une, voire deux pièces dans le cas de la pompe. Le processus de montage est simplifié par le pliage en Z, le clipage et le soudage aux ultrasons sur le moule d'injection. Ceci évite au maximum la manipulation de pièces séparées et toute capture de pièces en vrac lors de l'assemblage. Ce procédé de fabrication ne nécessite pas d'apport de matière. Il permet en outre d'intégrer facilement d'autres fonctionnalité sur la pièce moulée en trois partie, en utilisant la technique du surmoulage. Il est facile de faire l'étanchéité des entrées et des sorties de l'enceinte.

Bien que les exemples qui précèdent montrent la fixation des parties de paroi 1, 3 ; 11, 13 sur la partie intermédiaire 12, celle-ci pourrait aussi être noyée dans les surfaces périphériques d'assemblage homologues respectives de ces parties de paroi 1, 3 ; 11, 13, en sorte que cette partie intermédiaire est alors invisible et que l'on évite la double soudure.

Dans une variante, l'enceinte étanche selon les figures 1 à 4 pourrait être utilisée pour le conditionnement de deux substances, liquides, pâteuses ou pulvérulentes. A cet effet, la partie intermédiaire 2 doit être une cloison étanche pour diviser l'enceinte en deux compartiments. Ces substances pourraient être destinées à se combiner lors de leur utilisation.

## Revendications

1. Enceinte étanche dont la paroi est en deux parties (1, 3; 11, 13) qui présentent des surfaces périphériques d'assemblage homologues respectives (1a, 3a; 11a, 13a), réunies de manière étanche l'une à l'autre, cette enceinte comportant au moins une ouverture (6, 7; 16, 17) pour la relier avec l'extérieur et une partie intermédiaire (2; 12) disposée entre les deux parties de paroi (1, 3; 11, 13) de l'enceinte, **caractérisée en ce qu'**elle forme initialement une pièce unique avec la partie intermédiaire (2; 12) disposée entre les surfaces périphériques d'assemblage homologues (1a, 3a; 11a, 13a) des deux parties de paroi (1, 3; 11, 13), cette pièce unique comportant deux liaisons d'articulation (4, 5; 14, 15), entre deux portions opposées de la périphérie de ladite partie intermédiaire (2; 12) et respectivement les surfaces périphériques d'assemblage homologues (1a, 3a; 11a, 13a) des deux parties (1, 3; 11, 13) de la paroi de l'enceinte étanche.

2. Enceinte selon la revendication 1 dans laquelle la pièce unique dont est formée l'enceinte et sa partie intermédiaire est en matière plastique injectée.

3. Enceinte selon l'une des revendications précédentes dans laquelle chacune des deux parties de paroi (1, 3; 11, 13) présente une ouverture (6, 7; 16, 17) pour relier l'intérieur de l'enceinte avec l'extérieur.

4. Enceinte selon la revendication 3, dans laquelle une portion de la paroi de l'une desdites parties de paroi (1, 3; 11, 13) constitue une membrane de pompage (11a) et une portion de la partie intermédiaire (12) est conformée en clapet de contrôle (12b) de l'ouverture (16) de la partie de paroi (11) solidaire de ladite membrane de pompage (11a), en fonction des différences de pression induites par le déplacement de la membrane de pompage (11a) s'exerçant sur les deux faces du clapet de contrôle (12b), ladite partie intermédiaire (12) présentant une ouverture (12a) pour faire communiquer les volumes de l'enceinte situés des deux côtés de cette partie intermédiaire (12), un clapet de contrôle (20) de cette ouverture (12a) en fonction des différences de pression induites par le déplacement de la membrane de pompage (11a) étant positionné entre l'ouverture (12a) de ladite partie intermédiaire et l'ouverture (17) pour relier l'intérieur de l'enceinte avec l'extérieur de la partie de paroi (13) non munie de ladite membrane de pompage (11a).

5. Enceinte selon l'une des revendications 1 à 3, dans laquelle chacune desdites parties de paroi (1, 3) comporte une ouverture (6, 7) pour relier l'intérieur de l'enceinte avec l'extérieur, ladite partie intermédiaire (2) étant solidaire d'un filtre.

6. Enceinte selon l'une des revendications 1 à 3, dans laquelle ladite partie intermédiaire (2) sert de support pour un dispositif ou pour un circuit électronique.

7. Enceinte étanche selon la revendication 1 dans laquelle la partie intermédiaire (2) est une cloison étanche pour séparer l'enceinte en deux compartiments pour le conditionnement de deux substances.

8. Procédé de fabrication d'une enceinte selon la revendication 1, **caractérisé en ce qu'**on forme un moule en deux pièces (8, 9) en ménageant dans chacune d'elles une empreinte (8a, 9a) des faces externes des deux parties de paroi (1, 2; 11, 13) de l'enceinte et une empreinte (8b, 9b) des faces internes de ces mêmes deux parties de paroi (1, 2; 11, 13) de l'enceinte et on relie ces deux empreintes par une empreinte de ladite partie intermédiaire (2; 12) et desdites liaisons d'articulation (4, 5; 14, 15),
on réuni les deux pièces (8, 9) du moule et on injecte la matière plastique, on la refroidit et on sépare les deux pièces (8, 9) du moule,
on forme ensuite l'enceinte étanche en maintenant une des deux parties de paroi (1, 3; 11, 13) dans l'empreinte (8a, 9a) de sa face externe et on déplace l'autre des deux parties de paroi selon un mouvement de translation le long d'une trajectoire semi-circulaire dont le centre se situe sur la liaison pliable située entre la partie de paroi maintenue dans l'empreinte du moule et ladite partie intermédiaire (2; 12) et dont le rayon correspond à la distance séparant les deux liaisons d'articulation pour amener les surfaces périphériques d'assemblage homologues respectives (1a, 3a; 11a, 13a) des deux parties de paroi l'une contre l'autre avec interposition au moins partielle de la périphérie de ladite partie intermédiaire (2; 12) et on réalise une fixation étanche des surfaces d'assemblage périphériques homologues de ces deux parties de paroi (1, 3; 11, 13).

9. Procédé selon la revendication 8, selon lequel on forme une membrane de pompage (11a) et une ouverture d'admission (16), dans la paroi (11) de l'une des deux parties de paroi, une ouverture (12a) et un clapet (12b) de contrôle de l'ouverture d'admission (16) dans la partie intermédiaire (12) et on ménage un siège de positionnement (13a) d'un clapet (20) de contrôle de l'ouverture (12a) de la partie intermédiaire (12), situé entre cette partie intermédiaire (12) et une seconde ouverture (17), de refoulement, de l'autre partie de paroi (13) de l'enceinte.
